# FASCICULE DE BREVET EUROPEEN

(11) **EP 0 715 834 B1**
(45) Date de publication et mention de la délivrance du brevet: **27.03.2002**
(21) Numéro de dépôt: 95420332.9
(22) Date de dépôt: 28.11.1995
(51) Int. Cl.: A61F 2/06

(54) **Endoprothèse autoexpansible**
Selbstausspreizbare Endoprothese
Self-expanding endoprothesis

(30) Priorité: 09.12.1994 FR 9415085
(43) Date de publication de la demande: 12.06.1996
(73) Titulaire: SOFRADIM PRODUCTION, 01600 Trévoux (FR)
(72) Inventeur: Sgro Jean-Claude, 21000 Dijon (FR)
(74) Mandataire: Guerre, Dominique

(56) Documents cités:
- EP-A- 0 566 807
- WO-A-93/06883
- DE-A- 3 417 738
- DE-B- 1 766 921

## Description

La présente invention concerne une endoprothèse autoexpansible, c'est à dire une prothèse ou dispositif, de forme généralement tubulaire, destinée à être introduite et mise en place dans la lumière de tout canal ou conduit anatomique du corps humain ou animal, de manière à rétablir ou maintenir les dimensions normales et transversales de la dite lumière, par exemple pour rétablir ou maintenir le passage d'un fluide dans le dit canal ou conduit.

De telles prothèses endoluminales sont bien connues de la pratique médicale, dans les domaines de la chirurgie vasculaire, biliaire, ou intestinale, et sont souvent désignées sous le terme anglais de "stent".

Conformément au document EP-A-0 566 807, on a décrit et proposé une endoprothèse autoexpansible de construction monobloc ou monolithe, ayant une forme généralement cylindrique, ajourée, et ménageant une lumière intérieure. De manière générale, cette endoprothèse est susceptible de prendre deux conformations, l'une ramassée à l'encontre d'une force intrinsèque centrifuge de rappel élastique, résultant de la construction définie ci-après, dans laquelle la lumière intérieure présente un diamètre relativement faible, et l'autre déployée, obtenue par relâchement sous l'effet de cette même force centrifuge, dans laquelle la lumière intérieure précitée présente un diamètre relativement important.

Cette endoprothèse monobloc et ajourée comprend :
- 2 n éléments longitudinaux, dits axes externes, distribués parallèlement autour de l'axe de l'endoprothèse;
- 2 n autres éléments longitudinaux, dits axes intermédiaires, distribués parallèlement autour de l'axe de l'endoprothèse, entre les éléments longitudinaux externes, ou axes externes, n étant un nombre entier ;
- les éléments ou axes intermédiaires étant décalés longitudinalement par rapport aux éléments longitudinaux ou axes externes d'une même distance prédéterminée, de telle sorte qu'à chaque bout de l'endoprothèse les extrémités des éléments intermédiaires sont comprises dans un plan radial (perpendiculaires à l'axe de l'endoprothèse), séparé par la dite distance prédéterminée, du plan radial comprenant les extrémités voisines des éléments externes ;
- une pluralité d'anneaux, isolés quant à leur représentation à la figure 10 du document EP-A-0566807, distribués selon l'axe de l'endoprothèse, solidarisant les éléments ou axes longitudinaux, externes et intermédiaires, les uns des autres ; chaque anneau comprend, en suivant sa surface développée cylindrique, 2x2n brins transversaux reliés continûment les uns aux autres, formant deux à deux 2 n chevrons dont les sommets sont liés respectivement aux éléments ou axes intermédiaires, et les extrémités des branches sont liées respectivement aux éléments ou axes externes ; les chevrons des différents anneaux respectivement sont tous dirigés dans le même sens, de telle sorte qu'un élément ou axe longitudinal intermédiaire et tous les brins transversaux qui lui sont reliés de part et d'autre, présentent ensemble la forme d'une arête de poisson.

Grâce à la structure ou construction précédemment décrite, la conformation ramassée de l'endoprothèse est obtenue par traction longitudinale des éléments ou axes externes, par rapport aux éléments ou axes intermédiaires, dans la direction de l'axe de l'endoprothèse.

La présente invention a pour objet un mode d'exécution de l'endoprothèse définie précédemment de manière générale, conférant à cette dernière une grande souplesse de part et d'autre de son axe, lui permettant de s'adapter sans difficultés aux différentes formes ou angles, des conduits ou canaux anatomiques du corps humain ou animal. Et le mode d'exécution recherché ne doit pas compromettre la solidité ou résistance mécanique de l'endoprothèse, en particulier sans points de faiblesse dans cette dernière.

Selon la présente invention, les modalités pratiques suivantes sont retenues.

D'une part, les différents anneaux sont distincts les uns des autres, et des éléments longitudinaux externes et intermédiaires, et comportent chacun des brins transversaux, présentant ensemble la forme d'un fil essentiellement continu et fermé, conformé en zigzag, et compris dans une enveloppe fictive, par exemple cylindrique ; les sommets des angles sortants et rentrants des différents anneaux en zigzag sont alignés respectivement selon des lignes fictives externes et intermédiaires, parallèles à l'axe de l'endoprothèse. Et d'autre part, au moins une partie des éléments longitudinaux externes et intermédiaires sont des éléments filiformes, souples, entrecroisés avec les fils des différents anneaux respectivement, à l'endroit des lignes fictives externes et/ou intermédiaires ; et les dits éléments filiformes souples et/ou les fils des différents anneaux sont agencés pour être liés en position les un par rapports aux autres, à l'endroit de leurs entrecroisements respectifs.

La présente invention est maintenant décrite par référence au dessin annexé, dans lequel :
- la figure 1 représente une endoprothèse conforme à la présente invention, vue en perspective, et dans une conformation déployée ;
- la figure 2 représente une vue de dessus de l'endoprothèse représentée à la figure 1 ;
- la figure 3 représente, en vue développée et de côté, l'endoprothèse représentée aux figures 1 et 2 ;
- la figure 4 représente une vue partielle de côté de l'endoprothèse représentée aux figures 1 à 3 ;
- la figure 5 représente une vue développée partielle, de côté, d'un anneau appartenant à la prothèse selon les figures 1 à 4 ;
- la figure 6 représente une vue développée partielle, de côté, d'une endoprothèse conforme à un deuxième mode d'exécution de l'invention ;
- la figure 7 représente une vue partielle de côté, à l'échelle agrandie, de la prothèse représentée à la figure 6 ;
- la figure 8 représente une vue partielle de côté d'un anneau, réalisé selon un autre mode d'exécution de l'invention, et appartenant à une endoprothèse selon la figure 7.

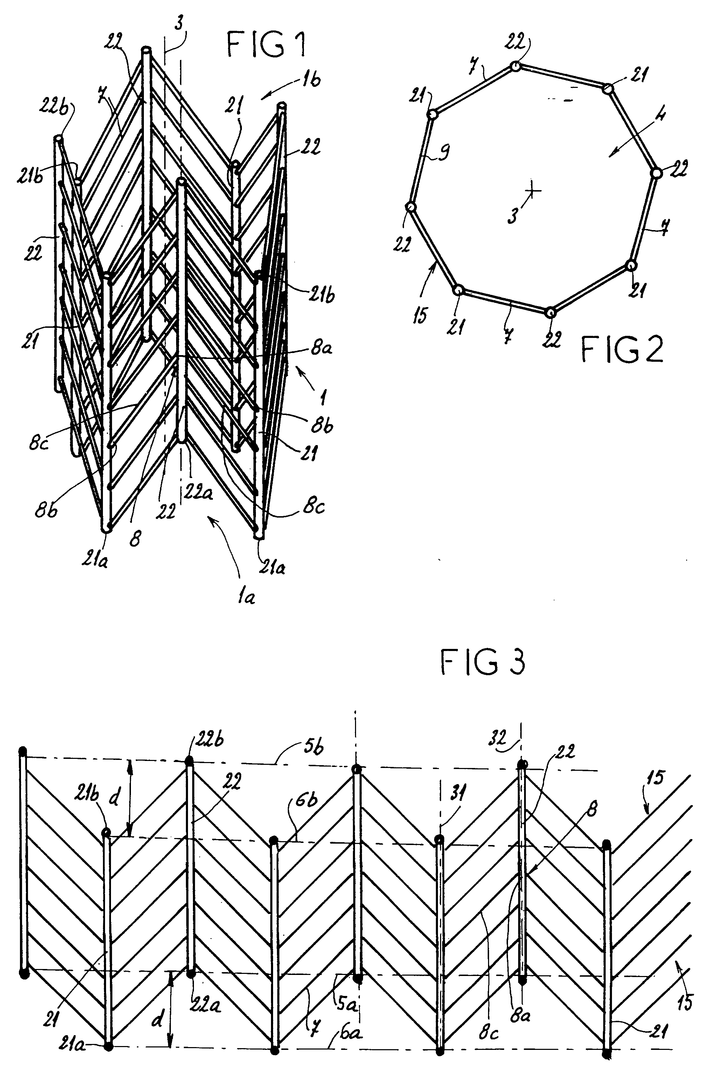

Conformément aux figures 1 à 5 et selon un premier mode d'exécution de l'invention, une endoprothèse 1 autoexpansible selon l'invention n'a pas une construction monobloc ou monolithe, puisque constituée de manière générale par différents anneaux 15, coaxiaux avec l'axe 3 de l'endoprothèse, distincts les uns des autres, et par des éléments longitudinaux, filiformes et souples, distincts des anneaux 15, parmi lesquels on distinguera pour les besoins de la description, des éléments externes 21 et des éléments intermédiaires 22.

Plus précisément, l'endoprothèse 1 comprend :
- 2 n éléments longitudinaux 21 externes, distribués parallèlement autour de l'axe 3 de l'endoprothèse ;
- 2 n autres éléments longitudinaux intermédiaires 22, distribués parallèlement autour de l'axe 3 de l'endoprothèse, et disposés entre les éléments externes 21, n étant un nombre entier, par exemple égal à 4 dans le cas de la représentation des figures 1 à 5 ;
- une pluralité d'anneaux 15, distribués selon l'axe 3 de l'endoprothèse, solidarisant ou reliant les éléments longitudinaux externes 21 et intermédiaires 22 les uns aux autres.

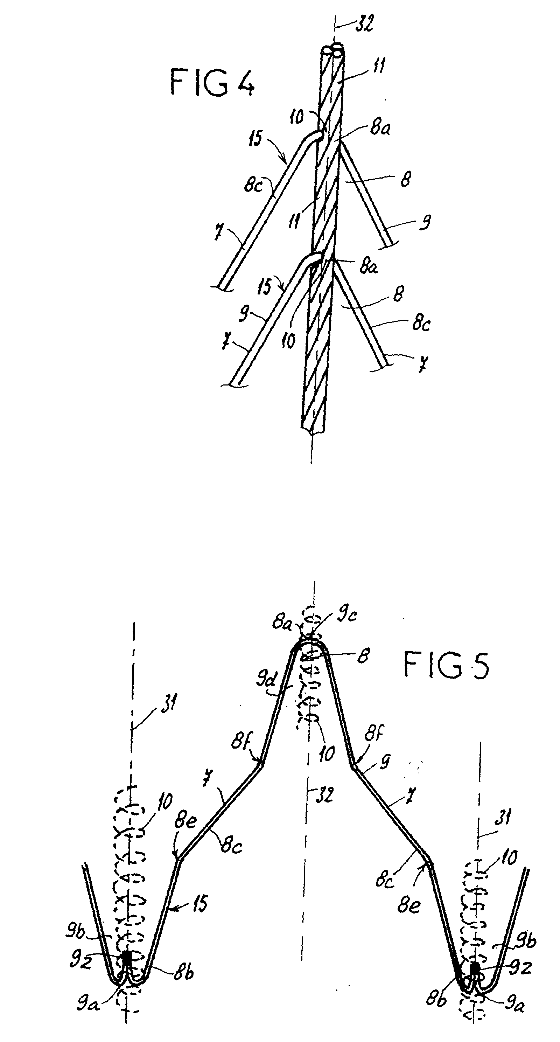

Comme mieux montré par la figure 3, les éléments longitudinaux intermédiaires 22 sont décalés longitudinalement, ou parallèlement à l'axe 3, par rapport aux éléments longitudinaux externes 21, de la même distance prédéterminée (d), de telles sorte qu'à chaque bout 1a ou 1b de l'endoprothèse, les extrémités 22a ou 22b des éléments intermédiaires 22 sont comprises dans un plan radial 5a ou 5b, séparé par la distance (d) du plan radial 6a ou 6b, comprenant les extrémités voisines 21a ou 21b, des éléments externes 21.

De manière générale, chaque anneau 15 comprend, en suivant sa surface développée cylindrique, 2x2 n brins 7 transversaux reliés continûment les uns aux autres, formant deux à deux 2 n chevrons 8, dont les sommets 8a sont liés respectivement aux éléments longitudinaux intermédiaires 22, et les extrémités 8b des branches 8c sont liées respectivement aux éléments longitudinaux externes 21. Les brins 7 transversaux de chaque anneau 15 ont ensemble la forme d'un fil 9 substantiellement continu et fermé, conformé en zigzag, et compris dans une enveloppe fictive de forme cylindrique. Les sommets 9a et 9c respectivement des angles sortants 9b et rentrants 9d des différents anneaux 15 sont alignés respectivement selon des lignes fictives externes 31 et intermédiaires 32, parallèles à l'axe 3 de l'endoprothèse. Les chevrons 8 des différents anneaux 15 respectivement sont tous dirigés dans le même sens, de telle sorte que, l'endoprothèse étant vue par l'extérieur et de côté, un élément longitudinal intermédiaire 22 et tous les brins transversaux qui lui sont reliés ou attachés (comme défini ci-après) de part et d'autres, présentent la forme d'une arête de poisson.

Comme représenté plus particulièrement aux figures 1 et 4, les éléments longitudinaux filiformes 21 et 22 sont entrecroisés avec les fils 9 des différents anneaux 15 respectivement, à l'endroit ou au niveau des lignes fictives externes 31 et intermédiaires 32, définies précédemment. Les éléments filiformes longitudinaux 21 et 22, et/ou les fils 9 des différents anneaux 15, sont agencés pour être liés en position définitive les uns par rapport aux autres, à l'endroit de leurs entrecroisements 10 respectifs. Pour ce faire, les éléments filiformes souples 21 et 22 consistent chacun en une pluralité de fils 11 torsadés ensemble, au travers desquels passent les fils 9 des différents anneaux 15, à l'endroit de leurs entrecroisements 10.

La structure précédemment décrite présente une force intrinsèque centrifuge de rappel élastique, du fait qu'elle résulte de l'assemblage en quelque sorte de plusieurs motifs en arête de poisson solidarisés les uns aux autres par les éléments longitudinaux externes et intermédiaires 21 et 22.

Par traction longitudinale exercée sur les éléments externes 21, par rapport aux éléments intermédiaires 22, selon la direction de l'axe 3 de l'endoprothèse, il est possible de faire passer cette dernière de sa conformation déployée et relâchée, dans laquelle la lumière intérieure 4 présente un diamètre relativement important, à une conformation ramassée, à l'encontre de la force centrifuge intrinsèque de rappel élastique, précédemment définie. En pratique, cette traction peut être obtenue, pour la mise en place de l'endoprothèse, par toutes sortes de dispositifs permettant son introduction dans un canal ou conduit anatomique, comme plus particulièrement représenté aux figures à 6 à 8 du document EP-A-0566807.

Par rapport à la construction précédemment définie, les modifications ou variantes suivantes doivent être apportées.

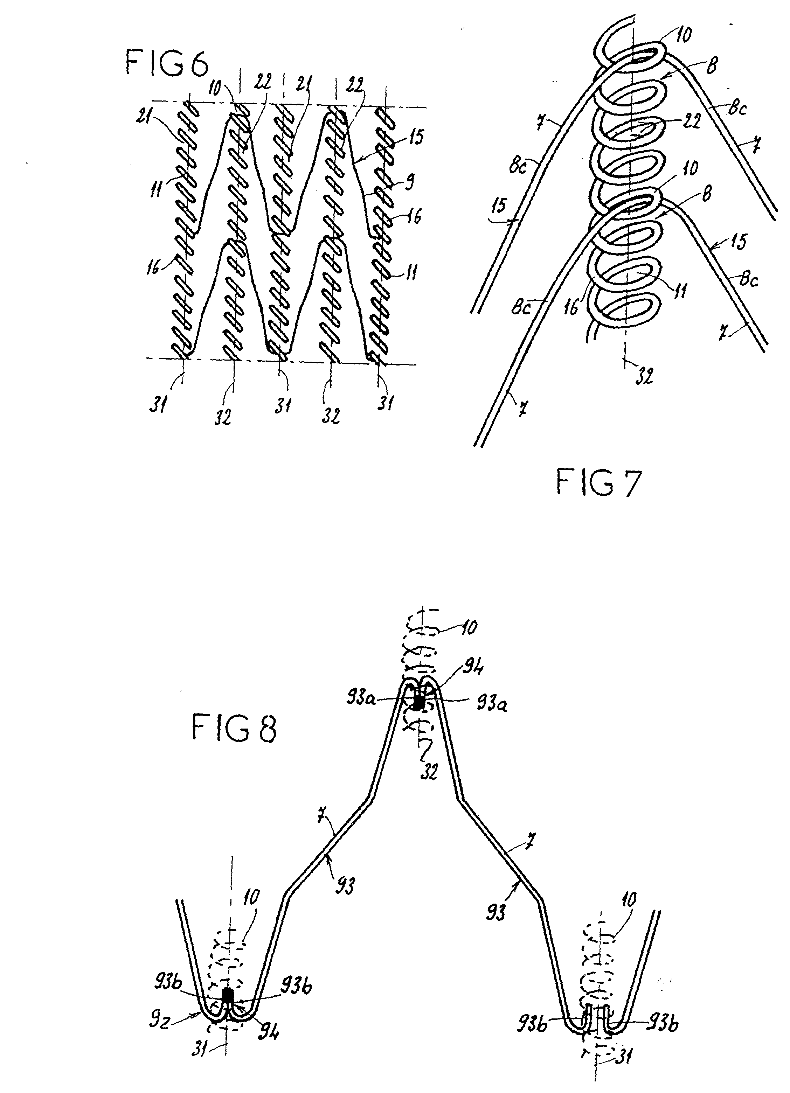

Conformément aux figures 6 et 7, les éléments filiformes et longitudinaux 21 et 22, souples, consistent chacun en au moins un fil en hélice, de telle sorte que, à l'endroit de leurs entrecroisements 10, les fils 9 des différents anneaux passent et sont retenus aux travers des hélices 11. Les éléments filiformes 21 et 22 peuvent être obtenus par l'association d'au moins deux hélices inversées, assemblées l'une sur l'autre,

Conformément à la représentation de la figure 3, les extrémités 21a, 21b, 22a et 22b des éléments filiformes souples 21 et 22 sont arrondies, par exemple selon un bout ayant la forme d'une goutte.

Plusieurs modalités différentes peuvent être mises en oeuvre pour obtenir chaque anneau 15 en zigzag :
- on peut tout d'abord former en zigzag une section de fil métallique, dont les deux extrémités sont ensuite solidarisées l'une à l'autre, par exemple par soudure ;
- conformément à la figure 5, on peut aussi découper dans du fil métallique une pluralité de sections 91, qui sont ensuite conformées en chevrons ; et ces derniers sont assemblés les uns aux autres, de manière à former un anneau 15 fermé en zigzag, par exemple par soudure des extrémités 8b contigües des chevrons 8, placés les uns à côté des autres, avec leurs sommets respectifs dirigés dans le même sens.
- conformément à la figure 8, le fil 9 de chaque anneau 15 est obtenu en découpant dans du fil, par exemple métallique, une pluralité de demi-sections 93 conformées chacune en demi-chevron, en assemblant les demi-sections les unes aux autres, de manière à former des chevrons complets, lesquels sont assemblés les uns aux autres de manière à former un anneau 15 fermé en zigzag, et ceci par exemple par soudure 94 des extrémités 93a, 93b, contigües des demi-chevrons 93 placés les uns à côté des autres, avec les sommets respectifs des chevrons 8, dirigés dans le même sens.

Les sommets 9a et 9c des anneaux 15 en zigzag peuvent être aplatis, et orientés, de manière perpendiculaire à l'axe 3 de l'endoprothèse.

Pour conférer encore plus de souplesse à l'endoprothèse, conformément à la représentation de la figure 5, les branches 8c des anneaux 15 en zigzag présentent chacune deux angles 8e et 8f obtus, l'un rentrant et l'autre sortant.

## Revendications

1. Endoprothèse (1) auto expansible destinée au maintien des parois de canaux anatomiques, de forme généralement cylindrique ajourée, ménageant une lumière (4) intérieure, susceptible de prendre deux conformations, l'une ramassée à l'encontre d'une force intrinsèque centrifuge de rappel élastique de l'endoprothèse, dans laquelle la lumière intérieure (4) présente un diamètre relativement faible et l'autre déployée, relâchée sous l'effet de ladite force centrifuge, dans laquelle la lumière intérieure (4) présente un diamètre relativement important, ladite endoprothèse comprenant :
- 2 n éléments longitudinaux (21) dits externes, distribués parallèlement autour de l'axe (3) de l'endoprothèse
- 2 n autres éléments longitudinaux (22) dits intermédiaires, distribués parallèlement autour de l'axe (3) de l'endoprothèse entre les éléments externes, n étant un nombre entier ;
- les éléments longitudinaux intermédiaires (22) étant décalés longitudinalement par rapport aux éléments longitudinaux externes (21) de la même distance prédéterminée (d), de telle sorte qu'à chaque bout (1a et 1b) de l'endoprothèse, les extrémités (22a,22b) des éléments intermédiaires sont comprises dans un plan radial (5a,5b), séparé par la dite distance prédéterminée (d) du plan radial (6a,6b) comprenant les extrémités voisines (21a, 21b) des éléments externes (21) ;
- une pluralité d'anneaux (15), distribués selon l'axe (3) de l'endoprothèse, solidarisant les éléments longitudinaux externes (21) et intermédiaires (22) les uns aux autres , chaque anneau (15) comprenant en suivant sa surface développée 2x2n brins (7) transversaux reliés continûment les uns aux autres, formant deux à deux 2n chevrons (8), dont les sommets (8a) sont liés respectivement aux éléments longitudinaux intermédiaires (22), et les extrémités (8b) des branches (8c) sont liées respectivement aux éléments longitudinaux externes (21), les chevrons (8) des différents anneaux (15) respectivement étant tous dirigés dans le même sens, de telle sorte qu'un élément longitudinal intermédiaire (22) et tous les brins transversaux (7) qui lui sont reliés, de part et d'autre, présentent la forme d'une arête de poisson ;
- la conformation ramassée étant obtenue par traction longitudinale des éléments externes (21), par rapport aux éléments intermédiaires (22), dans la direction de l'axe (3) de l'endoprothèse, **caractérisée en ce que**, d'une part les différents anneaux (15) sont distincts les uns des autres, et des éléments longitudinaux externes (21) et intermédiaires (22), et comportent chacun des brins (7) transversaux ayant ensemble la forme d'un fil (9) continu et fermé conformé en zigzag, et compris dans une enveloppe fictive des différents anneaux, les sommets (9a, 9c) des angles sortants (9b) et rentrants (9d) étant alignés respectivement selon des lignes fictives externes (31) et intermédiaires (32), parallèles à l'axe (3) de l'endoprothèse, et d'autre part, au moins une partie des dits éléments longitudinaux (21, 22) sont des éléments filiformes souples, entrecroisés avec les fils (9) des différents anneaux (15) respectivement, à l'endroit des lignes fictives externes (31) et/ou intermédiaires (32), et les dits éléments filiformes (21,22) et/ou les fils (9) des différents anneaux (15) sont agencés pour être liés en position, les uns par rapport aux autres, à l'endroit de leurs entrecroisements (10).

2. Endoprothèse selon revendication 1, **caractérisée en ce que** les éléments filiformes souples (21,22) consistent chacun en une pluralité de fils torsadés (11) au travers desquels passent les fils (9) des différents anneaux (15), à l'endroit de leurs entrecroisements (10).

3. Endoprothèse selon la revendication 1, **caractérisée en ce que** les éléments filiformes souples (21, 22) comprennent à l'endroit de leurs entrecroisements (10), des hélices (16) au travers desquelles passent les fils (9) des différents anneaux (15).

4. Endoprothèse selon la revendication 1, **caractérisée en ce que** les éléments filiformes souples (21, 22) consistent chacun en au moins un fil (11) en hélice.

5. Endoprothèse selon la revendication 1, **caractérisée en ce que** les extrémités (21a, 21b, 22a, 22b) des éléments filiformes souples (21, 22) sont arrondies, par exemple selon un bout ayant la forme d'une goutte.

6. Endoprothèse selon la revendication 1, **caractérisée en ce que** le fil (9) de chaque anneau (15) est obtenu en formant une section de fil, par exemple métallique, dont les deux extrémités sont ensuite solidarisées.

7. Endoprothèse selon la revendication 1, **caractérisée en ce que** le fil (9) de chaque anneau (15) est obtenu en découpant dans du fil, par exemple métallique, une pluralité de sections (91), en conformant chaque section en chevron (8), en assemblant les chevrons (8) les uns aux autres, de manière à former un anneau (15) fermé en zigzag, par exemple par soudure (92) des extrémités (8b) contigües des chevrons (8) placés les uns à côté des autres, avec leurs sommets respectifs dirigés dans le même sens.

8. Endoprothèse selon la revendication 1, **caractérisée en ce que** le fil (9) de chaque anneau (15) est obtenu en découpant dans du fil, par exemple métallique, une pluralité de demi-sections (93) conformées chacune en demi-chevron, en assemblant les demi-sections les unes aux autres, de manière à former des chevrons complets, lesquels sont assemblés les uns aux autres de manière à former un anneau (15) fermé en zigzag, et ceci par exemple par soudure (94) des extrémités (93a, 93b) contigües des demi-chevrons (93) placés les uns à côté des autres, avec les sommets respectifs des chevrons 8, dirigés dans le même sens.

9. Endoprothèse selon la revendication 1, **caractérisée en ce que** les sommets (9a, 9c) des anneaux (15) sont relativement aplatis et orientés de manière perpendiculaire à l'axe (3) de l'endoprothèse.

10. Endoprothèse selon la revendication 1, **caractérisée en ce que** les branches (8c) des anneaux (15) présentent chacune deux angles (8e, 8f) obtus, l'un rentrant et l'autre sortant.

## Patentansprüche

1. Selbst-expandierbare Endoprothese (1) von allgemein zylindrischer, durchbrochener Form zum Halten von Wänden anatomischer Kanäle, die ein inneres Lumen (4) ausbildet, das in der Lage ist, zwei Zustände einzunehmen, und zwar einen entgegen einer intrinsischen zentrifugalen elastischen Rückstellkraft gerafften Zustand, in dem das innere Lumen (4) einen relativ geringen Durchmesser aufweist, und einen ausgebreiteten, unter der Wirkung der zentrifugalen Kraft entspannten Zustand, in dem das innere Lumen (4) einen relativ großen Durchmesser aufweist, wobei die Endoprothese aufweist:
- 2n als äußere bezeichnete, längs verlaufende Elemente (21), die parallel um die Achse (3) der Endoprothese verteilt sind;
- 2n weitere, als dazwischenliegende bezeichnete, längsverlaufende Elemente (22), die parallel um die Achse (3) der Endoprothese zwischen den äußeren Elementen verteilt sind, wobei n eine ganze Zahl ist;
- wobei die dazwischenliegenden längsverlaufenden Elemente (22) in Längsrichtung bzgl. der äußeren längsverlaufenden Elemente (21) um denselben vorbestimmten Abstand (d) versetzt sind, derart, daß an beiden Enden (1a und 1b) der Endoprothese die äußeren Enden (22a, 22b) der dazwischenliegenden Elemente in einer radialen Ebene (5a, 5b) einbegriffen sind, die um den vorbestimmten Abstand (d) von der radialen Ebene (6a, 6b) getrennt ist, die die benachbarten äußeren Enden (21a, 21b) der äußeren Elemente (21) enthält;
- wobei eine Mehrzahl von Ringen (15), die entlang der Achse (3) der Endoprothese verteilt sind, die äußeren (21) und die dazwischenliegenden (22) längsverlaufenden Elemente miteinander vereinen, wobei jeder Ring (15) entsprechend seiner abgewickelten Oberfläche 2 x 2n querverlaufende Stückchen (7) aufweist, die ununterbrochen miteinander verbunden sind, und die paarweise 2n Spitzkörper (8) bilden, deren Spitzen (8a) jeweils mit den dazwischenliegenden längsverlaufenden Elementen (22) verbunden sind, und wobei die äußeren Enden (8b) der Zweige (8c) jeweils mit den äußeren längsverlaufenden Elementen (21) verbunden sind, wobei die Spitzkörper (8) der verschiedenen Ringe (15) jeweils in die gleiche Richtung gerichtet sind, derart, daß ein dazwischenliegendes längsverlaufendes Element (22) und alle querverlaufenden Stückchen (7), die mit diesem zu beiden Seiten verbunden sind, die Form eines Fischrückgrates aufweisen;
- wobei der geraffte Zustand durch längsgerichteten Zug der äußeren Elemente (21) relativ zu den dazwischenliegenden Elementen (22) in Richtung der Achse (3) der Endoprothese erhalten wird, **dadurch gekennzeichnet, daß**, einerseits, sich die verschiedenen Ringe (15) untereinander und von den äußeren (21) und dazwischenliegenden (22) längsverlaufenden Elementen unterscheiden und jeweils die querverlaufenden Stückchen (7) aufweisen, die zusammen die Form eines ununterbrochenen und geschlossenen Fadens (9) in Zick-Zack-Form aufweisen und in einer gedachten Einhüllenden der verschiedenen Ringe einbegriffen sind, wobei die Spitzen (9a, 9c) der ausgehenden (9b) und der eingehenden (9d) Winkel jeweils entlang gedachter äußerer (31) und dazwischenliegender (32), zur Achse (3) der Endoprothese paralleler Linien ausgerichtet sind, und daß, andererseits, zumindest ein Teil der genannten längsverlaufenden Elemente (21, 22) biegsame fadenförmige Elemente sind, die jeweils mit den Fäden (9) der verschiedenen Ringe (15) an der Stelle der gedachten äußeren (31) und/oder dazwischenliegenden (32) Linien verkreuzt sind, und daß die genannten fadenförmigen Elemente (21, 22) und/oder die Fäden (9) der verschiedenen Ringe (15) so ausgestaltet sind, daß sie an der Stelle ihrer Kreuzungspunkte (10) in einer Position relativ zueinander verbunden sind.

2. Endoprothese nach Anspruch 1, **dadurch gekennzeichnet, daß** die biegsamen fadenförmigen Elemente (21, 22) jeweils aus einer Mehrzahl gekordelter Fäden (11) bestehen, durch die die Fäden (9) der verschiedenen Ringe (15) an der Stelle ihrer Kreuzungspunkte (10) hindurchgehen.

3. Endoprothese nach Anspruch 1, **dadurch gekennzeichnet, daß** die biegsamen fadenförmigen Elemente (21, 22) an der Stelle ihrer Kreuzungspunkte Wendeln (16) aufweisen, durch die die Fäden (9) der verschiedenen Ringe (15) hindurchgehen.

4. Endoprothese nach Anspruch 1, **dadurch gekennzeichnet, daß** die biegsamen fadenförmigen Elemente (21, 22) jeweils aus zumindest einem Faden (11) in Wendelform bestehen.

5. Endoprothese nach Anspruch 1, **dadurch gekennzeichnet, daß** die äußeren Enden (21a, 21b, 22a, 22b) der biegsamen fadenförmigen Elemente (21, 22) abgerundet sind, beispielsweise entsprechend einem Ende, das die Form eines Tropfens aufweist.

6. Endoprothese nach Anspruch 1, **dadurch gekennzeichnet, daß** der Faden (9) jedes Ringes (15) durch Bilden eines beispielsweise metallischen Fadenabschnitts erhalten wird, dessen beide äußere Enden anschließend miteinander verbunden werden.

7. Endoprothese nach Anspruch 1, **dadurch gekennzeichnet, daß** der Faden (9) jedes Ringes (15) durch Ausschneiden einer Mehrzahl von Abschnitten (91) aus einem beispielsweise metallischen Faden erhalten wird, wobei jeder Abschnitt als Spitzkörper (8) ausgebildet wird, wobei die Spitzkörper (8) untereinander zusammengefügt werden, derart, daß ein geschlossener Ring (15) in Zick-Zack-Form gebildet wird, beispielsweise durch Schweißung (92) der aneinanderstoßenden äußeren Enden (8b) der mit ihren jeweiligen, in die gleiche Richtung zeigenden Spitzen nebeneinander angeordneten Spitzkörper (8).

8. Endoprothese nach Anspruch 1, **dadurch gekennzeichnet, daß** der Faden (9) jedes Ringes (15) durch Ausschneiden einer Mehrzahl von Halbabschnitten (93) aus einem beispielsweise metallischen Faden erhalten wird, die jeweils als Halbspitzkörper ausgebildet sind, wobei die Halbabschnitte untereinander zusammengefügt werden, derart, daß vollständige Spitzkörper gebildet werden, die derart untereinander zusammengefügt werden, daß ein geschlossener Ring (15) in Zick-Zack-Form gebildet wird, und zwar beispielsweise durch Schweißung (94) der aneinanderstoßenden äußeren Enden (93a, 93b) der nebeneinander angeordneten Halbspitzkörper (93), wobei die jeweiligen Spitzen der Spitzkörper (8) in dieselbe Richtung zeigen.

9. Endoprothese nach Anspruch 1, **dadurch gekennzeichnet, daß** die Spitzen (9a, 9c) der Ringe (15) relativ abgeflacht und rechtwinklig zur Achse (3) der Endoprothese orientiert sind.

10. Endoprothese nach Anspruch 1, **dadurch gekennzeichnet, daß** die Zweige (8c) der Ringe (15) jeweils zwei stumpfe Winkel (8e, 8f) aufweisen, und zwar einen eingehenden und einen ausgehenden Winkel.

## Claims

1. A self-expanding endoprosthesis (1) intended to support the walls of anatomical channels, being of a generally cylindrical form and of openwork design, forming an internal lumen (4), and being capable of assuming two configurations, namely one in which it is folded-down counter to an inherent centrifugal force of elastic return of the endoprosthesis, in which the internal lumen (4) exhibits a relatively small diameter, and the other in which it is deployed, released under the effect of said centrifugal force, in which the internal lumen (4) exhibits a relatively large diameter, said endoprosthesis comprising:
- 2n longitudinal elements (21), called external elements, distributed in a parallel arrangement about the axis (3) of the endoprosthesis;
- 2n other longitudinal elements (22), called intermediate elements, distributed in a parallel arrangement about the axis (3) of the endoprosthesis, between the external elements, n being an integer;
- the intermediate longitudinal elements (22) being offset longitudinally with respect to the external longitudinal elements (21) by the same predetermined distance (d), in such a way that, at each end (1a and 1b) of the endoprosthesis, the extremities (22a, 22b) of the intermediate elements are included in a radial plane (5a, 5b) separated by said predetermined distance (d) from the radial plane (6a, 6b) including the adjacent extremities (21a, 21b) of the external elements (21);
- a plurality of rings (15) distributed along the axis (3) of the endoprosthesis, joining the external (21) and intermediate (22) longitudinal elements to one another, each ring (15) comprising, along its developed surface, 2 x 2n transverse strands (7) connected continuously to one another and forming, in pairs, 2 n chevrons (8) whose vertices (8a) are linked respectively to the longitudinal intermediate elements (22) and the extremities (8b) of the arms (8c) are linked respectively to the longitudinal external elements (21), respectively the chevrons (8) of the various rings (15) being all oriented in the same direction, in such a way that one intermediate longitudinal element (22) and all the transverse strands (7) which are connected to it on either side together exhibit the shape of a fish bone;
- the folded-down configuration being obtained by longitudinal traction of the external elements (21) with respect to the intermediate elements (22), in the direction of the axis (3) of the endoprosthesis, **characterized in that**, on the one hand, the various rings (15) are distinct from one another, and from the external (21) and intermediate (22) longitudinal elements, and in each case comprise transverse strands (7), together having the form of a continuous and closed filament (9), of zigzag configuration, and included in an imaginary envelope of the various rings, the vertices (9a, 9c) of the salient (9b) and reentrant (9d) angles being aligned respectively along external (31) and intermediate (32) imaginary lines which are parallel to the axis (3) of the endoprosthesis, and, on the other hand, at least some of said longitudinal elements (21, 22) are flexible, filiform elements intersecting with the filaments (9) of the various rings (15) respectively, at the site of the external (31) and/or intermediate (32) imaginary lines, and said filiform elements (21, 22) and/or the filaments (9) of the various rings (15) being designed to be linked to one another in position, at the site of their respective intersections (10).

2. The endoprosthesis as claimed in claim 1, **characterized in that** the flexible filiform elements (21, 22) each consist of a plurality of twisted filaments (11) through which the filaments (9) of the various rings (15) pass at the site of their intersections (10).

3. The endoprosthesis as claimed in claim 1, **characterized in that** the flexible filiform elements (21, 22) include, at the site of their intersections (10), coils (16) through which the filaments (9) of the various rings (15) pass.

4. The endoprosthesis as claimed in claim 1, **characterized in that** the flexible filiform elements (21, 22) each consist of at least one helical filament (11).

5. The endoprosthesis as claimed in claim 1, **characterized in that** the extremities (21 a, 21 b, 22a, 22b) of the flexible filiform elements (21, 22) are rounded, for example as an end having the shape of a bead.

6. The endoprosthesis as claimed in claim 1, **characterized in that** the filament (9) of each ring (15) is obtained by forming a section of filament, for example a wire, the two extremities of which are then joined.

7. The endoprosthesis as claimed in claim 1, **characterized in that** the filament (9) of each ring (15) is obtained by cutting from a filament, for example a wire, a plurality of sections (91), shaping each section to form a chevron (8), and connecting the chevrons (8) to one another so as to form a closed zigzagged ring (15), for example by welding (92) the contiguous extremities (8b) of the chevrons (8), placed alongside one another, with their respective vertices oriented in the same direction.

8. The endoprosthesis as claimed in claim 1, **characterized in that** the filament (9) of each ring (15) is obtained by cutting from a filament, for example a wire, a plurality of half-sections (93) each configured as a half chevron, by connecting the half-sections to one another in such a way as to form complete chevrons which are connected to one another in such a way as to form a closed zigzagged ring (15), and this being done, for example, by welding (94) the contiguous extremities (93a, 93b) of the half chevrons (93), placed alongside one another, with the respective vertices of the chevrons (8) oriented in the same direction.

9. The endoprosthesis as claimed in claim 1, **characterized in that** the vertices (9a, 9c) of the rings (15) are relatively flattened and oriented perpendicular to the axis (3) of the endoprosthesis.

10. The endoprosthesis as claimed in claim 1, **characterized in that** the arms (8c) of the rings (15) each have two obtuse angles (8e, 8f), one reentrant and the other salient.
